# NOUVEAU FASCICULE DE BREVET EUROPEEN

(11) **EP 1 046 402 B2**
(45) Date de publication et mention de la décision concernant l'opposition: **04.04.2012**
(45) Mention de la délivrance du brevet: 22.08.2007
(21) Numéro de dépôt: 00401073.2
(22) Date de dépôt: 18.04.2000
(51) Int. Cl.: A61L 27/36, A61L 27/60, A61L 27/24, C12N 5/07

(54) **Equivalent de peau agée, son procédé de préparation et son utilisation**
Gealtelter Haut-äquivalent, deren Herstellungsverfahren und Verwendungen
Aged skin equivalent, methods of preparation and uses thereof

(30) Priorité: 20.04.1999 FR 9904970
(43) Date de publication de la demande: 25.10.2000
(73) Titulaire: L'Oréal, 75008 Paris (FR)
(72) Inventeur: Pageon, Hervé, 92800 Puteaux (FR); Asselineau, Daniel, 92160 Antony (FR); Tachon, Pierre, 92160 Antony (FR)
(74) Mandataire: Allab, Myriam

(56) Documents cités:
- EP-A- 0 789 074
- NEMECEK G, DAYAN A.: "Safety evaluation of human living skin equivalents" TOXICOLOGIC PATHOLOGY, vol. 27, no. 1, janvier 1999 (1999-01), pages 101-103, XP000856930
- REISER K. ET AL.: "Nonenzymatic glycation of type I collagen: the effects on aging on preferential glycation sites" JOURNAL OF BIOLOGICAL CHEMISTRY, vol. 267, no. 4, 1992, pages 24207-24216, XP002123914
- DEMARCHEZ M. ET AL.: "Migration of Langerhans cells into human epidermis of "reconstructed" skin, normal skin, or healing skin, after grafting onto the nude mouse" JOURNAL OF INVESTIGATIVE DERMATOLOGY, vol. 100, no. 5, 1993, pages 648-652, XP000856942
- OIMOMI M ET AL.: "The effect of fructose on collagen glycation" KOBE JOURNAL OF MEDICAL SCIENCES, vol. 35, no. 4, 1989, pages 195-200, XP000856925
- FONT J. ET AL.: "A new three-dimensional culture of human keratinocytes: optimization of differentiation" CELL BIOLOGY AND TOXICOLOGY, vol. 10, no. 5-6, 1994, pages 353-359, XP000856943
- HENDRIX S.W. ET AL.: "Differential response of basal keratinocytes in a human skin equivalent to ultraviolet irradiation" ARCHIVES OF DERMATOLOGICAL RESEARCH, vol. 290, no. 8, 1998, pages 420-424, XP000856944
- EIJI KAWANO ET AL.: 'Nonenzymatic Glycation Alters Properties of Collagen as a Substratum for Cells' MATRIX vol. 10, 1990, pages 300 - 305
- JACQUES FREY ET AL.: 'Effets de la glycation sur la fibrillogenèse du collagène de type I dans un modèle de tissu conjonctif' C. R. SOC. BIOL. vol. 187, 1993, pages 223 - 231

## Description

La présente invention concerne un nouvel équivalent de peau âgée, son procédé d'obtention ainsi que l'équivalent d'épiderme et l'équivalent de derme âgé compris dans cet équivalent de peau âgée et leurs utilisations in vitro.

On cherche à mettre au point, depuis plusieurs années, des modèles de peau reconstruite qui permettent d'effectuer les études nécessaires à la meilleure compréhension du rôle de la peau tant dans le domaine mécanique que dans le domaine physiologique.

Ainsi, des modèles plus ou moins proches de la peau humaine ont pu être mis au point. On peut citer par exemple les modèles décrits dans les demandes de brevets EP-A-285471, EP-A-285474, EP-A-789074, EP-A-502172, EP-A-418035, WO-A-9116010, EP-A-197090, EP-A-20753, FR-A-2665175, FR-A-2689904.
Des études ont également porté sur l'évaluation du pouvoir allergène des constituants d'un équivalent de peau humaine (Nemecek G et al., Toxicology Pathology, vol.27, n°1, janvier 1999, pages 101-103), sur la recolonisation par des cellules de Langerhans d'un équivalent de peau greffée sur une souris (Demarchez M et al., Journal of Investigative Dermatology, vol. 100, n° 5, 1993, pages 648-652) et sur l'expression différentielle de marqueurs de différenciation comme les intégrines dans les couches suprabasales de l'épiderme à partir d'un équivalent d'épiderme (Font et al., Cell Biology and toxicology, vol. 10, n°5-6, 1994, pages 353-359) ou d'un équivalent de peau humaine soumis à un rayonnement UV (Hendrix S.W et al., Archives of Dermatological Research, vol. 290, n°8, 1998, pages 420-424).De manière très générale, les modèles de peau reconstruite décrits dans ces documents comprennent des kératinocytes humains associés ou non à d'autres cellules de la peau comme les mélanocytes et/ou les cellules de Langerhans, déposés sur un support, souvent un équivalent de derme, et cultivés dans des conditions telles qu'ils entrent dans un programme de différenciation aboutissant à la formation d'un équivalent d'épiderme.

Les équivalents de derme décrits à ce jour sont soit des membranes artificielles comme par exemple les filtres de marque Millipore, des substituts sous-cutanés à base de collagène, du plastique ou tout autre support compatible avec la viabilité cellulaire, soit des supports plus élaborés pour les rendre plus proches du derme naturel, comme le derme préalablement désépidermisé ou des lattices mixtes collagène/fibroblastes.

Dans les lattices mixtes collagène/fibroblastes l'association de collagène natif et de fibroblastes humains isolés conduit à l'obtention d'un équivalent de derme mimant un derme qui n'a pas subi l'action du temps.

Les protocoles utilisés pour la préparation desdites lattices utilise du collagène provenant en général de tissus jeunes, collagène qui a cependant subi les importantes modifications post-traductionnelles intervenant dans les processus complexes mais néanmoins normaux de sa biosynthèse, mais qui n'a pas subi toutes les modifications pouvant intervenir particulièrement au cours du vieillissement.
On sait en particulier qu'au cours du vieillissement, ainsi qu'au cours de certaines maladies telle que le diabète, des processus non enzymatiques s'opèrent faisant intervenir un ose (glucose ou ribose) qui réagit selon la réaction de Maillard avec un groupement aminé (par exemple un résidu de lysine) du collagène pour former une base de Schiff. Celle-ci, après un réarrangement moléculaire dit d'Amadori, peut conduire, par une succession de réactions, à un pontage intramoléculaire comme par exemple de type pentosidine. Ce phénomène, dénommé glycation du collagène, augmente de façon régulière avec l'âge entraînant une augmentation régulière de la teneur de la peau en produits de glycation. Ces produits de glycation sont par exemple la pyrraline, la carboxyméthyl-lysine, la pentosidine, la crosslines, la N^{ε}(2-carboxyéthyl)-lysine (CEL), la glyoxal-lysine dimer (GOLD), la méthylglyoxal-lysine dimer (MOLD), la 3DG-ARG imidazolone, les versperlysines A, B, C, la thréosidine ou encore les produits finaux de glycosylation avancée (advanced glycosylation ends products ou AGEs). Reiser M. et al. (Journal of Biochemistry, vol 267, n°4, 1992, pages 24207-24216) a mis notamment en évidence la présence de sites préférentiels de glycation sur les chaînes de collagène, conservés au cours du vieillissement.
Ce phénomène est amplifié dans certaines maladies comme par exemple le diabète.
Sans vouloir introduire une quelconque théorie du vieillissement de la peau, il faut noter que d'autres caractéristiques qui pourraient également être une conséquence de ces phénomènes de glycation comme une diminution de la dénaturation par la chaleur, une augmentation de la résistance à la digestion enzymatique et une augmentation des pontages intermoléculaires, ont pu être mises en évidence au cours du vieillissement de la peau (Tanaka S. et col., 1988, J. Mol. Biol., 203, 495-505 ; Takahashi M. et col., 1995, Analytical Biochemistry, 232, 158-162). De plus, des modifications dues à la glycation de certains constituants de la membrane basale comme le collagène IV, la laminine et la fibronectine ont pu être mise en évidence (Tarsio JF. et col. , 1985, Diabetes, 34, 477-484 ; Tarsio JF. et col, 1988, Diabetes, 37, 532-539 ; Sternberg M. et col., 1995, C. R. Soc. Biol., 189, 967-985).
Ainsi on comprend qu'au cours du vieillissement de la peau les propriétés physico-chimiques du collagène se modifient et ce dernier devient plus difficilement soluble et plus difficilement dégradable.
Ainsi une des composantes de la peau âgée apparaît bien être le collagène glyqué.

On sait très bien que la peau résulte d'une étroite association entre au moins deux compartiments qui la constituent à savoir l'épiderme et le derme. Les interactions entre le derme et l'épiderme sont telles qu'il est raisonnable de penser qu'une modification de l'un peut avoir des conséquences sur l'autre. On peut suspecter que le vieillissement du derme en particulier avec ses phénomènes de glycation ne peut qu'avoir des conséquences sur l'épiderme qui lui est associé. Ainsi au cours du vieillissement cutané, la glycation du collagène doit entraîner des modifications de l'épiderme qui participent nécessairement au vieillissement de l'épiderme.
A ce titre la demanderesse a maintenant pu montrer qu'une protéine constitutive de l'épiderme normal, à savoir l'intégrine de type β1, récepteur de la matrice extracellulaire (voir Ruoslahti E., 1991, Cell biology of Extracellular Matrix, Plenum press New York, 343363), présente dans l'épiderme âgé une répartition de son expression très différente de ce qu'elle est dans un épiderme jeune. En effet, si dans un épiderme jeune, c'est à dire au sens de la demanderesse un épiderme de sujet jeune, cette protéine est exprimée dans les couches très inférieures de l'épiderme, à savoir au maximum jusqu'à la 2^{ième} couche suprabasale, il en est tout autrement dans un épiderme âgé, c'est à dire au sens de la demanderesse dans un épiderme de sujet âgé, où cette protéine est exprimée dans la plupart des couches de l'épiderme, jusque dans les dernières couches suprabasales, sous la couche cornée.

A ce jour aucun modèle de peau reconstruite in vitro n'est capable, soit à cause des protocoles de préparation utilisés, soit du simple fait qu'une fois reconstitué il ne subit pas de modification, de produire un équivalent de peau dont l'un au moins des constituants présente une des composantes du vieillissement cutané. Ainsi aucun modèle de peau reconstruite in vitro ne présente les caractéristiques d'une peau âgée et ne permet l'étude des processus qui y conduise, ni l'étude des composés et/ou compositions qui permettraient au moins de ralentir ce/ces processus. Les seules évaluations connues de ces phénomènes passent par des études in vivo, soit chez l'animal soit chez l'homme. Tout particulièrement, pour des raisons d'éthique, on comprend l'intérêt de disposer d'un tel modèle.

Des études sur la glycation sont connues dans l'art antérieur. Par exemple, il est décrit un procédé pour obtenir un équivalent de tissu conjonctif sous forme d'une lattice de collagène glyqué et de fibroblastes (voir à ce titre Frey et col. (1992, C. R. Soc. Biol., 187, 223-231). Cependant, outre que Frey et col. n'ont jamais cherché ni même suggéré qu'il soit possible de préparer à partir de leur lattice un équivalent de peau, ils n'ont jamais comparé celle-ci à un quelconque équivalent de derme. De plus, mais tout en reconnaissant la validité du modèle de tissu conjonctif de Frey et col., il faut bien admettre que le protocole utilisé par ces auteurs ne peut conduire aux objectifs que s'est fixés la demanderesse, à savoir reproduire in vitro une peau, et donc par voie de conséquence un épiderme et un derme, présentant tout ou partie des caractéristiques d'une peau âgée. En incubant le collagène et le sucre pendant 9 jours à une température de 4°C, Frey et col. initient la réaction de glycation du collagène, réaction qui se poursuit par la suite dans la lattice dans laquelle le collagène est ainsi préglyqué. Si l'on veut atteindre un taux de glycation suffisant, pour mimer une peau âgée, il est alors nécessaire de laisser se poursuivre la réaction de glycation dans la lattice ainsi formée, c'est à dire en cours de contraction, pendant encore un temps suffisant pour atteindre le taux recherché. Or l'homme du métier sait que pour établir un équivalent d'épiderme contenant au moins des kératinocytes sur un équivalent de derme de type lattice de collagène/fibroblaste, l'ensemencement des kératinocytes doit intervenir sur une lattice qui ne peut avoir dépassé un stade de contraction déterminée. Il ressort donc de ceci qu'il n'est pas possible d'obtenir par le protocole de Frey un équivalent de derme mimant un derme âgé voire très âgé, c'est à dire fortement voire très fortement glyqué.

Ainsi, à la connaissance de la demanderesse il n'a jamais été décrit l'obtention d'un équivalent de peau comprenant un équivalent d'épiderme obtenu sur un équivalent de derme âgé, particulièrement sur une lattice comprenant au moins du collagène glyqué et des fibroblastes.

On comprend alors le grand intérêt qu'il y a à disposer d'un modèle de peau reconstruite dont l'un au moins des composants présenterait un des aspects d'une peau âgée.

La demanderesse, qui depuis longtemps s'intéresse à la réalisation de modèle de peau reconstruite in vitro, a mis au point un nouveau modèle de peau reconstruite comprenant un équivalent d'épiderme et un équivalent de derme, caractérisé par le fait que ledit équivalent de derme comprend un équivalent de derme âgé, particulièrement une lattice comprenant au moins du collagène glyqué et des fibroblastes.

Ainsi un premier objet de l'invention est un nouvel équivalent de derme âgé, comprenant au moins du collagène glyqué et des fibroblastes et susceptible d'être obtenu par un procédé de préparation d'un équivalent de peau âgée comprenant un équivalent d'épiderme et un équivalent de derme âgé comprenant une lattice comprenant au moins du collagène glyqué et des fibroblastes, caractérisé par le fait que dans une première étape on prépare une lattice comprenant au moins du collagène glyqué et des fibroblastes, ledit collagène étant un mélange de collagène préglyqué et de collagène non-glyqué, le collagène étant glyqué préalablement à la formation de la lattice, et que dans une seconde étape on reconstitue sur la lattice obtenue à la première étape un équivalent d'épiderme comprenant au moins des kératinocytes, l'équivalent de derme âgé présentant un taux de glycation compris entre 2 et 30.

Différentes méthodes pour suivre la formation des produits de glycation sont décrites dans l'art antérieur. A cet égard on peut citer par exemple les méthodes décrites par Cefalu W.T. et col. (Journal of gerontology; Biological sciences, 1995, vol.50 A, n°6, 13337-13341), par Sell D. R. (Diabetes / Metabolism, 1991, vol 7, n°4, 239-251), par Miyata T. et col. (Journal of the american society of nephrology, 1996, vol.7, n°8, 1198-1206, ou encore par Furth A. J. (Analytical biochemistry, 1991, 192, 109-111. Ainsi, il est possible de mesurer le taux de composé glyquant lié au collagène et/ou le taux de composé glyquant restant après la réaction. Comme décrit précédemment la glycation du collagène conduit à la formation de produits de glycation comme par exemple la pyrraline, la carboxyméthyl-lysine, la pentosidine, la crosslines, la N^{ε}(2-carboxyéthyl)-lysine (CEL), la glyoxal-lysine dimer (GOLD), la méthylglyoxal-lysine dimer (MOLD), la 3DG-ARG imidazolone, les versperlysines A, B, C, la thréosidine ou encore les produits finaux de glycosylation avancée (advanced glycosylation ends products ou AGEs). Certains de ces produits de glycation présentent la particularité d'émettre après excitation une fluorescence mesurable. Par exemple la pentosidine, lorsque excitée à une longueur d'onde (λex) de 328 nm, émet une fluorescence à une longueur d'onde (λem) de 378 nm. De même, les AGEs lorsque excités à une longueur d'onde (λex) de 370 nm, émettent une fluorescence à une longueur d'onde (λem) de 440 nm. Le rapport de la fluorescence émise par un produit de glycation donné dans l'équivalent de derme comprenant au moins du collagène glyqué et des fibroblastes à la fluorescence émise par le même produit de glycation dans un équivalent de derme témoin comprenant au moins du collagène non glyqué et des fibroblastes, mesurée dans les mêmes conditions expérimentales, permet de caractériser le taux de glycation de l'équivalent de derme âgé.
Particulièrement, selon l'invention, on mesure la fluorescence de la pentosidine et/ou des AGEs tant dans l'équivalent de derme âgé comprenant du collagène glyqué que dans un témoin comprenant du collagène non glyqué.
Ainsi, selon l'invention l'équivalent de derme âgé présente un taux de glycation compris entre 2 et 30, et particulièrement compris entre 8 et 18.

Selon l'invention, le collagène peut être tout type de collagène de toute origine. A cet égard, on se référera aux différents types de collagène cités dans les revues de Van der Rest et Garonne, 1990, Biochem., vol. 72, 473-484 ou encore 1991, Faseb journal, vol.5, 2814-2823. Ainsi, selon l'invention, le collagène est choisi de préférence parmi les collagènes fibrillaires de type I, III ou V.

Préférentiellement selon l'invention on utilise du collagène d'origine animal, particulièrement du collagène d'origine bovine.
Le collagène préférentiellement utilisé selon l'invention est du collagène de type I. Très préférentiellement selon l'invention on utilise du collagène bovin de type I.

Bien entendu selon l'invention, on peut utiliser un mélange de collagène de différents types en toute proportion et/ou de différentes origines.

Selon l'invention, les fibroblastes peuvent provenir de toutes origines, mais sont préférentiellement des fibroblastes d'origine humaine. Ils peuvent être préparés selon n'importe quelle méthode connue de l'art antérieur, par exemple par dissociation mécanique et/ou enzymatique des macromolécules de la matrice extracellulaire du derme ou par croissance des fibroblastes à partir d'explants.

Bien entendu, l'équivalent de derme de l'invention comprend au moins du collagène glyqué et des fibroblastes mais peut également contenir tout autre composant qu'il pourrait être intéressant d'y introduire comme par exemple des cellules endothéliales, des macrophages ou encore des cellules nerveuses.

Un second objet de l'invention est un équivalent d'épiderme comprenant au moins des kératinocytes, caractérisé par le fait qu'il est susceptible d'être obtenu par ensemencement d'au moins des kératinocytes sur un équivalent de derme âgé comprenant au moins du collagène glyqué et des fibroblastes, tel que défini plus haut, et présentant une expression de l'intégrine β1 dans les cellules d'au moins les trois premières couches suprabasales.

On a vu précédemment dans le texte qu'en fonction de l'âge, certains marqueurs de l'épiderme pouvaient subir des modifications soit dans leur quantité soit dans la localisation de leur expression. Particulièrement la demanderesse a montré que l'expression de l'intégrine β1 dans l'épiderme est modifiée dans sa localisation avec l'âge de l'épiderme. En effet, si dans un épiderme jeune cette protéine est exprimée strictement dans au maximum les deux premières couches suprabasales, avec l'âge cette expression, tout en conservant sa localisation dans les deux premières couches suprabasales, apparaît dans les couches de plus en plus supérieures jusqu'à apparaître dans l'ensemble des couches suprabasales y compris les dernières couches jouxtant le stratum corneum.

Ainsi, selon l'invention, l'équivalent d'épiderme est caractérisé par le fait qu'il présente une expression de l'intégrine β1 modifiée, particulièrement une expression de l'intégrine β1 dans les cellules d'au moins les trois premières couches suprabasales.

Très préférentiellement selon l'invention, l'équivalent d'épiderme comprenant au moins des kératinocytes est caractérisé par le fait qu'il présente une expression de l'intégrine β1 modifiée, particulièrement une expression de l'intégrine β1 dans les cellules d'au moins les trois premières couches suprabasales et par le fait qu'il est obtenu par ensemencement d'au moins des kératinocytes sur un équivalent de derme comprenant au moins du collagène glyqué et des fibroblastes.

Bien évidemment toute méthode permettant de mettre en évidence l'expression de l'intégrine β1 est utilisable pour caractériser l'épiderme de l'invention. A titre d'exemple on peut citer le marquage à l'aide d'anticorps anti-intégrine β1.

Les kératinocytes utilisés selon l'invention peuvent provenir de toutes origines, mais sont préférentiellement des kératinocytes d'origine humaine. Ils peuvent être préparés selon n'importe quelle méthode connue de l'art antérieur. On citera à titre d'exemple la culture à partir d'épiderme dissocié provenant de prélèvement de peau normale ou la culture de kératinocytes issus de la gaine du follicule pileux.
De préférence selon l'invention on utilise des kératinocytes de peau humaine normale.

Préférentiellement, selon l'invention les kératinocytes utilisés sont préparés à partir d'épiderme humain dissocié provenant de prélèvement de peau humaine normale selon la méthode décrite dans Régnier et collaborateurs, Frontier of Matrix Biology, Vol.9, 4-35 (Karger, Basel 1981).

L'équivalent d'épiderme de l'invention comprend au moins de kératinocytes mais il peut comprendre tout autre type cellulaire qui pourrait y être incorporé comme par exemple des cellules de Langerhans et/ou des précurseurs de cellules de Langerhans et/ou des mélanocytes.
Bien entendu, l'équivalent de peau qui présente la meilleure similitude avec la peau normale est l'équivalent de peau qui contient les trois types cellulaires essentiels présents dans la peau normale.

Ainsi, avantageusement, le modèle de peau reconstruite selon l'invention comprend en outre des mélanocytes et/ou des cellules de Langerhans et/ou des précurseurs des cellules de Langerhans.

Les mélanocytes utilisés selon l'invention peuvent être isolés à partir de tout organe en contenant comme par exemple la peau normale ou le follicule de cheveu.

Préférentiellement, on utilise des mélanocytes isolés à partir de peau normale.

Toute méthode de préparation des mélanocytes connue de l'art antérieur peut être utilisée selon l'invention. On peut citer par exemple la méthode décrite dans Olsson et collaborateurs, Acta Derm. Venereol., 1994, 74, 226-268.

Les cellules de Langerhans et/ou les précurseurs de cellules de Langerhans utilisables selon l'invention peuvent être tels que décrits par la demanderesse dans sa demande de brevet européen publiée sous le numéro EP-A-789074.

Un troisième objet de l'invention est un équivalent de peau âgée, caractérisé par le fait qu'il comprend au moins un équivalent d'épiderme et un équivalent de derme âgé.

Un équivalent de peau âgée très préférentiel selon l'invention comprend un équivalent d'épiderme comprenant au moins des kératinocytes présentant une expression de l'intégrine β1 modifiée, particulièrement une expression de l'intégrine β1 dans les cellules d'au moins les trois premières couches suprabasales et est caractérisé par le fait qu'il est susceptible d'être obtenu par ensemencement d'au moins des kératinocytes sur un équivalent de derme âgé comprenant au moins du collagène glyqué et des fibroblastes, ledit équivalent de derme âgé présentant un taux de glycation compris entre 2 et 30, et particulièrement compris entre 8 et 18.

Un quatrième objet de l'invention est un procédé de préparation d'un équivalent de peau âgée, comprenant un équivalent d'épiderme et un équivalent de derme lui-même comprenant une lattice comprenant au moins du collagène glyqué et des fibroblastes, caractérisé par le fait que dans une première étape on prépare une lattice comprenant au moins du collagène glyqué et des fibroblastes ledit collagène étant un mélange de collagène pré-glyqué et de collagène non glyqué, le collagène étant glyqué préalablement à la formation de la lattice et que dans une seconde étape on reconstitue sur la lattice obtenue à la première étape un équivalent d'épiderme comprenant au moins des kératinocytes.

La première étape peut être réalisée par tout procédé connu de l'art antérieur, pour peu que le collagène utilisé puisse être glyqué préalablement.
Selon l'invention, on prépare la lattice en utilisant du collagène préalablement glyqué.
Préférentiellement selon l'invention on prépare la lattice selon la méthode décrite par Asselineau et col., 1987, (Models in dermato., vol.III, Ed. Lowe & Maibach, 1-7) en utilisant de collagène préglyqué.

Toute méthode connue de glycation peut être utilisée pour obtenir du collagène glyqué. On citera par exemple les méthodes décrites par Tanaka et col. (J. Mol. Biol., 1988, 203, 495-505), par Tarsio JF. et col. (1985, Diabetes, 34, 477-484), par Tarsio JF. et col. (1988, Diabetes, 37, 532-539) ou encore par Frey J. et col. (1992, C. R. Soc. Biol., 187, 223-231).

Préférentiellement selon l'invention, la glycation peut être obtenue par mise en présence d'une solution d'au moins un collagène et d'une solution d'au moins un agent glyquant de façon à induire la réaction de glycation du collagène in-vitro en l'absence de cellules.

Comme indiqué précédemment, le collagène utilisé peut être tout type de collagène, de toute origine, seul ou en mélange.
Préférentiellement selon l'invention on utilise du collagène d'origine animal, particulièrement du collagène d'origine bovine.
Le collagène préférentiellement utilisé selon l'invention est du collagène de type I. Très préférentiellement selon l'invention on utilise du collagène bovin de type I.

La solution de collagène peut être à une concentration comprise entre 2 mg/ml et 6 mg/ml et de préférence entre 3 mg/ml et 5 mg/ml.

L'agent glyquant peut être tout agent permettant la glycation c'est à dire capable de réagir selon la réaction de Maillard avec un groupement aminé du collagène pour former une base de Schiff. A cet égard on peut citer par exemple certains intermédiaires de la réaction de Maillard comme par exemple la glucosone, la 3-déoxyglucosone, le glyoxal, le méthyl-glyoxal ou encore les sucres.
Tout type de sucre est utilisable selon l'invention, qu'il soit sous forme monomérique ou polymérique. Selon l'invention, on utilise préférentiellement un sucre monomérique.
Par sucre on entend selon l'invention les produits qui possèdent plusieurs fois une fonction alcool avec au moins une fonction aldéhyde. On citera particulièrement les oses.
Parmi les sucres utilisables selon l'invention on peut citer entre autres le ribose, le fructose, ou le glucose. Préférentiellement selon l'invention on utilise du ribose ou du glucose.

Le sucre peut être dans l'une quelconque des conformations dextrogyre ou lévogyre. Préférentiellement selon l'invention, on utilise un sucre en conformation dextrogyre.
Particulièrement selon l'invention on utilise du D-fructose, du D-ribose ou du D-glucose. Préférentiellement on utilise du D-ribose ou du D-glucose.

Bien entendu on peut utiliser l'agent glyquant seul ou en mélange.

La quantité d'agent glyquant utilisable selon l'invention doit être suffisante pour permettre le démarrage des réactions non enzymatiques conduisant à la formation de base de Schiff. On comprend que la variation de cette quantité permet d'obtenir un produit final, le collagène glyqué, dont le taux de glycation varie de très peu glyqué à très fortement glyqué. Ainsi la quantité d'agent glyquant peut être comprise entre 0,5% et 20% préférentiellement entre 1% et 10% en poids du poids total de la solution de collagène.

La réaction de glycation s'effectue à une température proche de la température ambiante. Ainsi la réaction s'effectue à une température comprise entre 15°C et 30°C, préférentiellement entre 20°C et 25°C.
La durée de la réaction de glycation est fonction du taux de glycation recherché. On comprend que plus le temps est long plus le taux de glycation est élevé. Ainsi la durée de la réaction de glycation est comprise entre 15 jours et 2 mois, préférentiellement entre 25 et 35 jours.
Lorsque l'on choisi de réaliser la glycation préalablement à la préparation de la lattice, il est possible de faire subir au collagène glyqué toutes les étapes subséquentes nécessaires à l'obtention d'un produit le plus pur possible. Ainsi il est possible de chercher à éliminer toute trace d'agent glyquant qui n'aurait pas réagit au cours de la réaction. On peut pour cela utiliser toute technique connue. Par exemple on fait subir à la solution de collagène préglyqué une série de dialyses contre de l'eau et/ou de l'acide acétique.

Selon une variante de l'invention, la solution de collagène «préglyqué» obtenue peut être mélangée à du collagène natif avant utilisation pour la préparation de l'équivalent de derme. Dans ce cas le rapport du collagène glyqué au collagène non glyqué peut être compris entre 25 et 75 et préférentiellement entre 45 et 55. La variation de ce rapport permet de moduler le taux de glycation de la lattice.
Selon l'invention on utilise un mélange de collagène glyqué et de collagène non glyqué, et encore plus préférentiellement un mélange de collagène glyqué et de collagène non glyqué dans un rapport 50/50.

La seconde étape du procédé de l'invention peut être réalisée par tout procédé connu de l'art antérieur.
A cet égard on citera par exemple les méthodes décrites dans les demandes de brevets EP-A-285471, EP-A-285474, EP-A-789074, EP-A-502172, EP-A-418035, WO-A-9116010, EP-A-197090, EP-A-20753, FR-A-2665175, FR-A-2689904 ou encore celle décrite par Asselineau et col., 1985, (Exp. Cel. Res., 536-539) et 1987, (Models in dermato., vol.III, Ed. Lowe & Maibach, 1-7).
Préférentiellement selon l'invention, on utilise la méthode décrite par Asselineau et collaborateur.

Les kératinocytes utilisés selon l'invention peuvent provenir de toutes origines, mais sont préférentiellement des kératinocytes d'origine humaine. Ils peuvent être préparés selon n'importe quelle méthode connue de l'art antérieur. On citera à titre d'exemple la culture à partir d'épiderme dissocié provenant de prélèvement de peau normale ou la culture de kératinocytes issus de la gaine du follicule pileux.

Préférentiellement, selon l'invention les kératinocytes utilisés sont préparés à partir d'épiderme humain dissocié provenant de prélèvement de peau humaine normale selon la méthode décrite dans Régnier et collaborateurs, Frontier of Matrix Biology, Vol.9, 4-35 (Karger, Basel 1981).
Avantageusement, après ensemencement des kératinocytes sur le support, la culture peut être maintenue immergée dans un milieu nutritif qui peut être par exemple le milieu décrit par Rheinwald et Green, 1975, (Cell, 6, (3), 317-330) milieu qui permet la prolifération des kératinocytes (nommé par ailleurs dans le texte milieu 3F).

Après un temps d'incubation de 3 à 15 jours, préférentiellement de 7 à 9 jours, l'équivalent de peau est maintenu à l'interface air/liquide par exemple par dépôt sur une grille métallique. Le liquide est alors préférentiellement constitué du même milieu nutritif que le précédent.

L'incubation se poursuit ensuite jusqu'à obtention d'un équivalent de peau présentant les caractéristiques d'une peau, à savoir le support sur lequel se trouve un équivalent d'épiderme présentant les quatre types de couches cellulaires classiques, à savoir les couches basale, suprabasale, granuleuse et cornée.

Ainsi, l'incubation se poursuit pendant une durée comprise entre 5 et 30 jours , préférentiellement entre 7 et 10 jours.

Le modèle de peau reconstruite ainsi réalisé comprend de deux entités, le support et l'équivalent d'épiderme, qu'il est possible de séparer physiquement l'un de l'autre.
L'équivalent d'épiderme peut alors être utilisé séparément du support.

On a vu précédemment dans le texte qu'à ce jour aucun modèle de peau reconstruite in vitro ne présentait les caractéristiques d'une peau âgée et ne permettait l'étude des processus qui y conduise, ni l'étude des composés et/ou compositions qui permettraient au moins d'en modifier le processus. L'équivalent de peau obtenu selon l'invention résout ses problèmes, puisqu'il présente au moins une des caractéristiques de la peau âgée à savoir un collagène glyqué. Cette importante caractéristique que le modèle et son procédé permettent de faire varier en toute proportion, permet l'étude du phénomène de glycation en lui même ainsi que des modulateurs (inhibiteurs ou activateurs) de ce phénomène, l'étude des phénomènes liés à la peau âgée comme par exemple les rides, l'étude des modulateurs (composés isolés et/ou compositions) de l'apparition des rides (inhibiteurs particulièrement), l'étude du photovieillissement et de l'effet des rayonnements ultraviolets sur la peau ainsi que des modulateurs de ces effets (composés et/ou compositions de protections, filtres, etc.), l'étude de l'influence de la glycation sur les composants de la peau (poils et/ou cheveux, vaisseaux sanguins, fibres nerveuses, etc.), et dans le domaine thérapeutique l'étude des complications causées par le diabète via la glycation.

Ainsi l'invention a également pour objet l'utilisation in vitro d'un équivalent de peau âgée et/ou de derme âgé et/ou d'un équivalent d'épiderme âgé tels que décrits précédemment pour l'étude du phénomène de glycation en lui même ainsi que des modulateurs (inhibiteurs ou activateurs) de ce phénomène, l'étude des phénomènes liés à la peau et/ou l'épiderme âgé comme par exemple les rides, l'étude des modulateurs (composés isolés et/ou compositions) de l'apparition des rides (inhibiteurs particulièrement), l'étude du photovieillissement et de l'effet des rayonnements ultraviolets sur la peau et/ou le derme et/ou l'épiderme ainsi que des modulateurs de ces effets (composés et/ou compositions de protections, filtres, etc.), l'étude de l'influence de la glycation sur les composants de la peau et/ou du derme et/ou de l'épiderme (poils et/ou cheveux, vaisseaux sanguins, fibres nerveuses, etc.), et dans le domaine thérapeutique l'étude des complications causées par le diabète via la glycation.

On également vu que selon l'invention, l'équivalent d'épiderme reconstruit sur l'équivalent de derme comprenant au moins du collagène glyqué et des fibroblastes peut présenter une distribution de l'expression de l'intégrine β1 modifiée. Dans ce cas, si la variation de la distribution de ce marqueur est reliée à l'âge de l'épiderme, il est envisageable d'utiliser l'équivalent de peau âgée de l'invention pour évaluer tout produit apte à traiter la peau âgée en mesurant son effet par l'effet qu'il produit sur la modification de la distribution de l'expression de l'intégrine β1 dans l'équivalent d'épiderme.

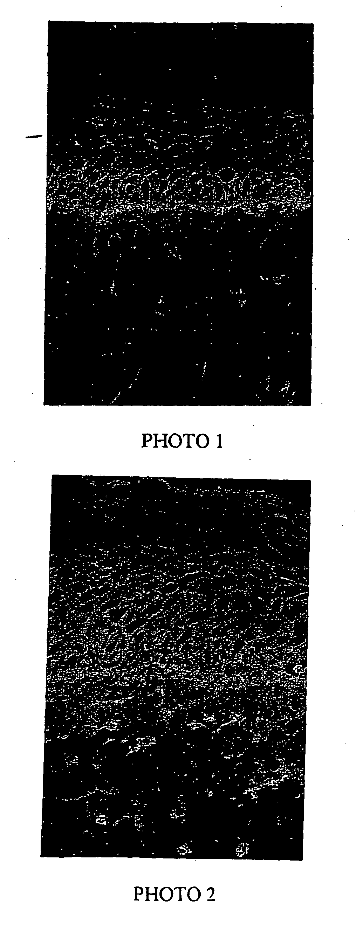

La figure 1 permet de mieux illustrer l'invention, sans toutefois en limiter sa portée. Dans cette figure, les photos montrent des coupes d'équivalents de peau après immunomarquage à l'aide d'un anticorps anti-intégrine β1. La photo 1 représente l'immunomarquage d'un équivalent de peau comprenant un équivalent d'épiderme reconstruit sur un équivalent de derme préparé avec du collagène non glyqué. La photo 2 représente l'immunomarquage d'un équivalent de peau comprenant un équivalent d'épiderme reconstruit sur un équivalent de derme préparé avec du collagène glyqué selon l'invention.
On va maintenant donner des exemples qui illustrent l'invention sans la limiter aucunement.

### Exemples :

Milieux et tampons sauf indication contraire, l'ensemble des milieux et tampons utilisés dans les exemples sont décrits dans Bell et col. 1979, (P.N.A.S. USA, 76, 1274-1278), Asselineau et Prunieras, 1984, (British J. of Derm., 111, 219-222) ou Asselineau et col., 1987, (Models in dermato., vol.III, Ed. Lowe & Maibach, 1-7).

### Exemple 1 : Préparation de collagène I bovin glyqué :

Dans un tube Falcon 50 ml on introduit 10 ml de solution de collagène I bovin à la concentration de 3mg/ml, 0,8 ml de soude 0,5N pour neutraliser la solution acide de collagène et 100 µl d'une solution de D-ribose à 1 M dans l'eau.
Le tube rendu opaque à la lumière est placé à l'horizontal et agité doucement à température ambiante (25°C) pendant 1 mois.
A la fin de la « préglycation », la solution est placée dans un boudin de dialyse (Spectra / Poly labo 32 mm n° 132655 / 85716) et subit une série de dialyses successives :
24 h contre de l'eau déminéralisée à 4°C afin d'éliminer le sucre non fixé ou les produits de dégradation du collagène ;
7 jours contre de l'acide acétique 0,5 N afin de remettre en solution le collagène en 2 bains de 3 jours et demi ;
3 x 24 h contre de l'acide acétique 0,017 N avec changement de bain chaque jour.
Après la dernière dialyse, le contenu du boudin de dialyse est récupéré dans un Bêcher stérile et la solution est transférée dans un tube Falcon de 50 ml stérile, rendu opaque à la lumière.
La solution de collagène « préglyquée » est alors prête à l'emploi. Elle peut être conservée à 4°C.

### Exemple 2 : Préparation d'un équivalent de derme âgé :

Dans un tube Falcon stérile, on introduit 3,22 ml de milieu MEM 1,76 X, 0,63 ml de sérum de veau foetal, 0,35 ml de soude 0,1 N, et 0,20 ml d'un mélange milieu MEM/Hépes contenant 10 % sérum de veau foetal (MEM/Hepes/SVF10).
On ajoute alors 0,50 ml milieu MEM/Hepes/SVF10 contenant des fibroblastes issus de plasties mammaires humaines préalablement préparés selon la méthode décrite par Bell et col. 1979, (P.N.A.S. USA, 76, 1274-1278), Asselineau et Prunieras, 1984, (British J. of Derm., 111, 219-222) ou Asselineau et col., 1987, (Models in dermato., vol.III, Ed. Lowe & Maibach, 1-7), à la concentration de 1x10⁶ cellules pour 0,5 ml de milieu de culture.

On ajoute alors lentement, contre la paroi du tube de façon à observer l'apparition d'un nuage blanchâtre, 2 ml d'un mélange volume/volume de collagène préglyqué de l'exemple 1 et de collagène non-glyqué ayant servi à la préparation du collagène préglyqué de l'exemple 1 à la concentration de 3 mg/ml dans de l'acide acétique au 1/1000. L'ensemble est alors mélangé avec précaution et déposé dans une boite de Pétri de diamètre 60 mm (type Falcon 60 mm, réf. 1016). La boite de Pétri est alors placée dans une étuve à 37°C et laissée environ 2h30. Lorsque l'on observe l'apparition de 2 phases, (gel + milieu), on désolidarise avec précaution la lattice de son support et l'on laisse la lattice ainsi désolidarisée de son support pendant 4 jours à l'étuve.

### Exemple 3 : mesure du taux de glycation de l'équivalent de derme âgé de l'exemple 2 :

Parallèlement à la réalisation de l'équivalent de derme âgé de l'exemple 2, un équivalent de derme sans collagène glyqué (mais avec du collagène non glyqué) est réalisé. Cet équivalent servira de témoin dans la détermination du taux de glycation de l'équivalent de derme glyqué de l'exemple 2.

2 lattices (une âgée, une témoin) préparées selon l'exemple 2 sont rincées trois fois au tampon phosphate salins (PBS) puis sont séchées. Les lattices sont alors placées dans un tube Eppendorf et soumises à une digestion à la pepsine (Sigma P-6887) à 37°C au bain-marie pendant une nuit (12 heures) à raison de 500 µg de pepsine par lattice dans 0,5 ml d'acide acétique 0,5 N.
On ajoute alors dans chaque tube 515 µl de soude 0,5 N et l'on filtre le contenu de chaque tube sur filtre spin x 0,22µm (Sigma).
La fluorescence est alors mesurée à l'aide d'un spectrofluorimètre de marque Hitachi, modèle F2000.
On mesure ainsi après excitation à λex = 328 nm la fluorescence émise à λem = 378 nm par la pentosidine et après excitation à λex = 370 nm la fluorescence émise à λem = 440 nm par les AGEs.

Les résultats obtenus sont présentés dans le tableau suivant :

| | Pentosidine | AGEs |
|---|---|---|
| Témoin | 650 | 430 |
| Lattice glyquée | 6100 | 1820 |

Dans cet exemple le taux de glycation de l'équivalent de derme âgé s'établit donc à 9,4 pour la pentosidine et à 4 pour les AGEs.

### Exemple 4 : Préparation d'un équivalent de peau âgée :

Un équivalent de derme âgé tel que préparé dans l'exemple 2 est montée bien étalée dans une boite de culture de type Corning ⌀ 60 mm sur une goutte de "colle" au collagène (0,6 ml) puis maintenue à 37°C dans une étuve pendant 20 - 30 minutes.
Un anneau en acier stérile est placé sur la lattice et 0,5 ml d'une suspension cellulaire de kératinocytes humains provenant de plasties mammaires préparés selon Régnier et collaborateurs, (Frontier of Matrix Biology, Vol.9, 4-35, Karger, Basel 1981), à raison de 100 000 cellules/ml en milieu MEM 10% FCS + 3F sont placés à l'intérieur de l'anneau.
Environ 6 ml de milieu (MEM 10% FCS + 3F) sont placés autour de l'anneau et la boite est placée dans une étuve à 37°C pendant 2 heures. L'anneau est alors retiré et la boite replacée à l'étuve.
Après 8 jours, la culture est alors placée à l'interface air/liquide, ledit liquide étant alors constitué du même milieu que précédemment.
La culture est ensuite poursuivie pendant 1 semaine jusqu'à obtention d'un équivalent d'épiderme histologiquement satisfaisant, c'est à dire un équivalent d'épiderme présentant les quatre couches cellulaires classiques, à savoir les couches basale, suprabasale, granuleuse et cornée.

### Exemple 5 : caractérisation de l'expression de l'intégrine β1 dans l'équivalent d'épiderme obtenu dans l'exemple 4 :

L'expression de l'intégrine β1 dans l'équivalent d'épiderme obtenu à l'exemple 4 est observée après immunomarquage à l'aide d'un anticorps monoclonal de souris dirigé contre l'intégrine β1 (Immunotech, Marseille, France, Cat.1151):
Après congélation, les équivalents de peau obtenu à l'exemple 4 sont découpés en lamelles de 5 µm d'épaisseur à l'aide d'un cryostat (marque/modèle) Les coupes sont alors rincées deux fois au PBS et 25 µl d'anticorps antiintègrine β1 dilué au 1/50 (Immunotech, Marseille, France, Cat.1151) sont déposés sur chaques coupes et laissés durant 30 minutes à la température ambiante (25°C). Les coupes sont alors rincées deux fois au PBS et 25 µl d'anticorps conjugués FITC (Rabbit anti mouse FITC, Dako F232), sont déposés sur chaques coupes et laissés pendant 30 minutes à la température ambiante (25°C). Les coupes sont rincées deux fois au PBS et observées après montage au microscope à fluorescence de marque LEICA, modèle LEITZ DMRB.

L'observation montre que dans le témoin l'intégrine β1 est exprimée dans la couche basale de l'épiderme reconstruit sur l'équivalent de derme non glyqué, ainsi que dans la première couche suprabasale (figure 1, photo 1), alors qu'elle est exprimée dans la totalité des couches suprabasales, jusque sous le stratum corneum dans l'épiderme reconstruit sur l'équivalent de derme âgé (figure 1, photo 2).
Ce résultats corrèlent avec l'observation d'une distribution in vivo de l'expression de l'intégrine β1 dans une peau de sujet jeune dans la couche basale et la première couche suprabasale et dans la couche basale et dans au moins les 3 premières couches suprabasales dans la peau d'un sujet âgé.

## Revendications

1. Procédé de préparation d'un équivalent de peau âgée comprenant un équivalent d'épiderme et un équivalent de derme âgé comprenant une lattice comprenant au moins du collagène glyqué et des fibroblastes, **caractérisé par le fait que** dans une première étape on prépare une lattice comprenant au moins du collagène glyqué et des fibroblastes, ledit collagène étant un mélange de collagène préglyqué et de collagène non-glyqué, le collagène étant glyqué préalablement à la formation de la lattice, et que dans une seconde étape on reconstitue sur la lattice obtenue à la première étape un équivalent d'épiderme comprenant au moins des kératinocytes.

2. Procédé selon la revendication 1, **caractérisé par le fait que** la glycation est obtenue par mise en présence d'une solution d'au moins un collagène et d'une solution d'au moins un agent glyquant de façon à induire la réaction de glycation in-vitro en l'absence de cellules.

3. Procédé selon l'une quelconque des revendications 1 ou 2, **caractérisé par le fait que** le collagène est du collagène d'origine humaine ou animale.

4. Procédé selon la revendication 3, **caractérisé par le fait que** le collagène est du collagène d'origine animale.

5. Procédé selon l'une quelconque des revendications 1 à 3, **caractérisé par le fait que** le collagène est du collagène d'origine bovine.

6. Procédé selon l'une quelconque des revendications 1 à 5, **caractérisé par le fait que** le collagène est choisi parmi les collagènes de type I, III, ou V.

7. Procédé selon l'une quelconque des revendications 1 à 6, **caractérisé par le fait que** le collagène est du collagène de type I.

8. Procédé selon l'une quelconque des revendications 1 à 7, **caractérisé par** la fait que le collagène est du collagène bovin de type I.

9. Procédé selon l'une quelconque des revendications 1 à 8, **caractérisé par le fait que** le collagène est à une concentration comprise entre 2 mg/ml et 6 mg/ml et de préférence entre 3 mg/ml et 5 mg/ml.

10. Procédé selon l'une quelconque des revendications 1 à 9, **caractérisé par le fait que** l'agent glyquant est un agent permettant la glycation c'est à dire capable de réagir selon la réaction de Maillard avec un groupement aminé du collagène pour former une base de Schiff.

11. Procédé selon la revendication 10, **caractérisé par le fait que** l'agent glyquant est choisi parmi la glucosone, la 3-déoxyglucosone, le glyoxal, le méthyl-glyoxal ou encore les sucres.

12. Procédé selon la revendication 11, **caractérisé par le fait que** l'agent glyquant est un sucre.

13. Procédé selon la revendication 12, **caractérisé par le fait que** le sucre est choisi parmi les oses.

14. Procédé selon la revendication 13, **caractérisé par le fait que** l'ose est choisi parmi le ribose, le fructose ou le glucose.

15. Procédé selon l'une quelconque des revendications 1 à 14, **caractérisé par le fait que** l'agent glyquant est choisi parmi le ribose ou le glucose.

16. Procédé selon l'une quelconque des revendications 1 à 15, **caractérisé par le fait que** la quantité d'agent glyquant est comprise entre 0,5% et 20% préférentiellement entre 1% et 10% en poids du poids total de la solution de collagène.

17. Procédé selon l'une quelconque des revendications 1 à 16, **caractérisé par le fait que** la réaction de glycation s'effectue à une température comprise entre 15°C et 30°C, préférentiellement entre 20°C et 25°C.

18. Procédé selon l'une quelconque des revendications 1 à 17, **caractérisé par le fait que** la durée de la réaction de glycation est comprise entre 15 jours et 2 mois, préférentiellement entre 25 et 35 jours.

19. Procédé selon l'une quelconque des revendications 1 à 18, **caractérisé par le fait que** le rapport du collagène glyqué au collagène non glyqué est compris entre 25 et 75 et préférentiellement entre 45 et 55.

20. Procédé selon l'une quelconque des revendications 1 à 19, **caractérisé par le fait que** les kératinocytes utilisés selon l'invention sont d'origine humaine.

21. Equivalent de derme âgé, suscpetible d'être obtenu par le procédé selon l'une des revendications 1 à 20, comprenant au moins du collagène glyqué et des fibroblastes, **caractérisé par le fait qu'**il présente un taux de glycation compris entre 2 et 30.

22. Equivalent de derme âgé selon la revendication 21, **caractérisé par le fait qu'**il présente un taux de glycation compris entre 8 et 18.

23. Equivalent de derme âgé selon l'une quelconque des revendications 21 ou 22, **caractérisé par le fait que** le collagène glyqué est d'origine animale ou humaine.

24. Equivalent de derme âgé selon l'une quelconque des revendications 21 à 23, **caractérisé par le fait que** le collagène glyqué est d'origine animale.

25. Equivalent de derme âgé selon l'une quelconque des revendications 21 à 24, **caractérisé par le fait que** le collagène glyqué est d'origine bovine.

26. Equivalent de derme âgé selon l'une quelconque des revendications 21 à 25, **caractérisé par le fait que** le collagène glyqué est du collagène de type I.

27. Equivalent de derme âgé selon l'une quelconque des revendications 21 à 26, **caractérisé par le fait que** les fibroblastes sont d'origine humaine.

28. Equivalent d'épiderme comprenant au moins des kératinocytes, **caractérisé par le fait qu'**il est susceptible d'être obtenu par ensemencement d'au moins des kératinocytes sur un équivalent de derme tel que défini dans l'une quelconque des revendications 21 à 27, et en ce qu'il présente une expression de l'intégrine β1 dans les cellules d'au moins les trois premières couches suprabasales.

29. Equivalent d'épiderme selon la revendication 28 , **caractérisé par le fait que** les kératinocytes sont d'origine humaine.

30. Equivalent d'épiderme selon l'une quelconque des revendications 28 ou 29, **caractérisé par le fait qu'**il comprend en outre des mélanocytes et/ou des cellules de Langerhans et/ou des précurseurs des cellules de Langerhans.

31. Equivalent de peau âgée, **caractérisé par le fait qu'**il comprend au moins un équivalent d'épiderme et un équivalent de derme âgé tel que décrit dans l'une quelconque des revendications 21 à 27.

32. Equivalent de peau âgée, selon la revendication 31, **caractérisé par le fait qu'**il comprend un équivalent d'épiderme tel que décrit dans l'une quelconque des revendications 28 à 30.

33. Utilisation in vitro d'un équivalent de derme âgé tel que décrit dans l'une quelconque des revendications 21 à 27, pour la préparation d'équivalent d'épiderme et/ou de peau âgée.

34. Utilisation *in vitro* d'un équivalent de derme âgé tel que décrit dans l'une quelconque des revendications 21 à 27, pour l'étude du phénomène de glycation en lui même ainsi que des modulateurs (inhibiteurs ou activateurs) de ce phénomène, l'étude du photovieillissement et de l'effet des rayonnements ultraviolets sur le derme ainsi que des modulateurs de ces effets (composés et/ou compositions de protections, filtres, etc.), ou l'étude de l'influence de la glycation sur les composants du derme.

35. Utilisation *in vitro* d'un équivalent d'épiderme tel que décrit dans l'une quelconque des revendications 28 à 30, pour l'étude du phénomène de glycation en lui même ainsi que des modulateurs (inhibiteurs ou activateurs) de ce phénomène, l'étude des phénomènes liés à l'épiderme âgé comme par exemple les rides, l'étude des modulateurs (composés isolés et/ou compositions) de l'apparition des rides (inhibiteurs particulièrement), l'étude du photovieillissement et de l'effet des rayonnements ultraviolets sur l'épiderme ainsi que des modulateurs de ces effets (composés et/ou compositions de protections, filtres, etc.), l'étude de l'influence de la glycation sur les composants de l'épiderme et les annexes de la peau.

36. Utilisation *in vitro* d'un équivalent de peau âgée tel que décrit dans l'une quelconque des revendications 31 ou 32 pour l'étude du phénomène de glycation en lui même ainsi que des modulateurs (inhibiteurs ou activateurs) de ce phénomène, l'étude des phénomènes liés à la peau et/ou l'épiderme âgé comme par exemple les rides, l'étude des modulateurs (composés isolés et/ou compositions) de l'apparition des rides (inhibiteurs particulièrement), l'étude du photovieillissement et de l'effet des rayonnements ultraviolets sur la peau et/ou le derme et/ou l'épiderme ainsi que des modulateurs de ces effets (composés et/ou compositions de protections, filtres, etc.), l'étude de l'influence de la glycation sur les composants et/ou les annexes de la peau et/ou du derme et/ou de l'épiderme (poils et/ou cheveux, vaisseaux sanguins, fibres nerveuses, etc.), et dans le domaine thérapeutique l'étude des complications causées par le diabète via la glycation.

## Claims

1. Method for preparing an aged skin equivalent comprising an epidermis equivalent and an aged dermis equivalent which comprises a lattice comprising at least glycated collagen and fibroblasts, **characterized in that** in a first step a lattice comprising at least glycated collagen and fibroblasts is prepared, the said collagen being a mixture of preglycated collagen and non-glycated collagen, the collagen being glycated prior to the formation of the lattice, and in a second step an epidermis equivalent comprising at least keratinocytes is reconstructed on the lattice obtained in the first step.

2. Method according to Claim 1, **characterized in that** the glycation is obtained by bringing together a solution of at least one collagen and a solution of at least one glycating agent in such a way as to induce the glycation reaction in vitro in the absence of cells.

3. Method according to either one of Claims 1 and 2, **characterized in that** the collagen is collagen of human or animal origin.

4. Method according to Claim 3, **characterized in that** the collagen is collagen of animal origin.

5. Method according to any one of Claims 1 to 3, **characterized in that** the collagen is collagen of bovine origin.

6. Method according to any one of Claims 1 to 5, **characterized in that** the collagen is chosen from the type I, III or V collagens.

7. Method according to any one of Claims 1 to 6, **characterized in that** the collagen is type I collagen.

8. Method according to any one of Claims 1 to 7, **characterized in that** the collagen is bovine type I collagen.

9. Method according to any one of Claims 1 to 8, **characterized in that** the collagen is at a concentration between 2 mg/ml and 6 mg/ml and preferably between 3 mg/ml and 5 mg/ml.

10. Method according to any one of Claims 1 to 9, **characterized in that** the glycating agent is an agent which enables the glycation, i.e. which is capable of reacting according to the Maillard reaction with an amino group of the collagen to form a Schiffs base.

11. Method according to Claim 10, **characterized in that** the glycating agent is chosen from glucosone, 3-deoxyglucosone, glyoxal, methylglyoxal or alternatively sugars.

12. Method according to Claim 11, **characterized in that** the glycating agent is a sugar.

13. Method according to Claim 12, **characterized in that** the sugar is chosen from oses.

14. Method according to Claim 13, **characterized in that** the ose is chosen from ribose, fructose or glucose.

15. Method according to any one of Claims 1 to 14, **characterized in that** the glycating agent is chosen from ribose or glucose.

16. Method according to any one of Claims 1 to 15, **characterized in that** the amount of glycating agent is between 0.5% and 20%, preferably between 1% and 10% by weight of the total weight of the collagen solution.

17. Method according to any one of Claims 1 to 16, **characterized in that** the glycation reaction is carried out at a temperature between 15°C and 30°C, preferably between 20°C and 25°C.

18. Method according to any one of Claims 1 to 17, **characterized in that** the duration of the glycation reaction is between 15 days and 2 months, preferably between 25 and 35 days.

19. Method according to any one of Claims 1 to 18, **characterized in that** the ratio of glycated collagen to non-glycated collagen is between 25 and 75 and preferably between 45 and 55.

20. Method according to any one of Claims 1 to 19, **characterized in that** the keratinocytes used according to the invention are of human origin.

21. Aged dermis equivalent, which can be obtained by the method according to any one of Claims 1 to 20, comprising at least glycated collagen and fibroblasts, **characterized in that** it has a level of glycation between 2 and 30.

22. Aged dermis equivalent according to Claim 21, **characterized in that** it has a level of glycation between 8 and 18.

23. Aged dermis equivalent according to either one of Claims 21 and 22, **characterized in that** the glycated collagen is of animal or human origin.

24. Aged dermis equivalent according to any one Claims 21 to 23, **characterized in that** the glycated collagen is of animal origin.

25. Aged dermis equivalent according to any one of Claims 21 to 24, **characterized in that** the glycated collagen is of bovine origin.

26. Aged dermis equivalent according to any one of Claims 21 to 25, **characterized in that** the glycated collagen is type I collagen.

27. Aged dermis equivalent according to any one of Claims 21 to 26, **characterized in that** the fibroblasts are of human origin.

28. Epidermis equivalent comprising at least keratinocytes, **characterized in that** it can be obtained by seeding at least keratinocytes on to a dermis equivalent as defined in any one of Claims 21 to 27, and **in that** it has expression of β1 integrin in the cells of at least the first three suprabasal layers.

29. Epidermis equivalent according to Claim 28, **characterized in that** the keratinocytes are of human origin.

30. Epidermis equivalent according to either one of Claims 28 and 29, **characterized in that** it comprises, in addition, melanocytes and/or Langerhans cells and/or precursors of Langerhans cells.

31. Aged skin equivalent, **characterized in that** it comprises at least one epidermis equivalent and one aged dermis equivalent as described in any one of Claims 21 to 27.

32. Aged skin equivalent according to Claim 31, **characterized in that** it comprises an epidermis equivalent as described in any one of Claims 28 to 30.

33. *In vitro* use of an aged dermis equivalent as described in any one of Claims 21 to 27, for the preparation of epidermis and/or aged skin equivalent.

34. *In vitro* use of an aged dermis equivalent as described in any one of Claims 21 to 27, for the study of the glycation phenomenon in itself and modulators (inhibitors or activators) of this phenomenon, the study of photoageing and the effect of ultraviolet rays on the dermis as well as modulators of these effects (protective compounds and/or compositions, screening agents, etc.), or the study of the influence of glycation on the components of the dermis.

35. *In vitro* use of an epidermis equivalent as described in any one of Claims 28 to 30, for the study of the glycation phenomenon in itself and modulators (inhibitors or activators) of this phenomenon, the study of phenomena linked to aged epidermis such as, for example, wrinkles, the study of modulators (isolated compounds and/or compositions) of the appearance of the wrinkles (particularly the inhibitors), the study of photoageing and the effect of ultraviolet rays on the epidermis as well as modulators of these effects (protective compounds and/or compositions, screening agents, etc.), the study of the influence of glycation on the components of the epidermis and the annexes of the skin.

36. *In vitro* use of an aged skin equivalent as described in either one of Claims 31 and 32, for the study of the glycation phenomenon in itself and modulators (inhibitors or activators) of this phenomenon, the study of phenomena linked to aged skin and/or epidermis such as, for example, wrinkles, the study of modulators (isolated compounds and/or compositions) of the appearance of the wrinkles (particularly the inhibitors), the study of photoageing and the effect of ultraviolet rays on the skin and/or the dermis and/or the epidermis as well as modulators of these effects (protective compounds and/or compositions, screening agents, etc.), the study of the influence of glycation on the components and/or appendages of the skin and/or dermis and/or epidermis (body hair and/or head hair, blood vessels, nerve fibres, etc.), and in the therapeutic domain the study of the complications caused by diabetes via glycation.

## Patentansprüche

1. Verfahren zur Herstellung eines Äquivalents der Altershaut, das ein Äquivalent der Epidermis und ein Äquivalent der Alters-Dermis umfasst, das ein Gerüst aufweist, das zumindest glykiertes Collagen und Fibroblasten aufweist, **dadurch gekennzeichnet, dass**
in einem ersten Schritt ein Gerüst hergestellt wird, das zumindest glykiertes Collagen und Fibroblasten enthält, wobei das Collagen ein Gemisch aus vorglykiertem Collagen und nichtglykiertem Collagen ist und vor der Erzeugung des Gerüsts glykiert wird, und **dadurch**, dass
in einem zweiten Schritt auf dem im ersten Schritt erhaltenen Gerüst ein Äquivalent der Epidermis aufgebaut wird, das zumindest Keratinocyten enthält.

2. Verfahren nach Anspruch 1, **dadurch gekennzeichnet, dass** die Glycation durch Inkontaktbringen einer Lösung mindestens eines Collagens mit einer Lösung mindestens eines Glycationsmittels in der Weise erzielt wird, dass die Glycation in vitro in Abwesenheit von Zellen induziert wird.

3. Verfahren nach Anspruch 1 oder 2, **dadurch gekennzeichnet, dass** das Collagen ein Collagen menschlichen oder tierischen Ursprungs ist.

4. Verfahren nach Anspruch 3, **dadurch gekennzeichnet, dass** das Collagen ein Collagen tierischen Ursprungs ist.

5. Verfahren nach einem der Ansprüche 1 bis 3, **dadurch gekennzeichnet, dass** das Collagen ein Rindercollagen ist.

6. Verfahren nach einem der Ansprüche 1 bis 5, **dadurch gekennzeichnet, dass** das Collagen unter den Collagenen vom Typ I, III oder V ausgewählt wird.

7. Verfahren nach einem der Ansprüche 1 bis 6, **dadurch gekennzeichnet, dass** das Collagen ein Collagen vom Typ I ist.

8. Verfahren nach einem der Ansprüche 1 bis 7, **dadurch gekennzeichnet, dass** das Collagen ein Rindercollagen vom Typ I ist.

9. Verfahren nach einem der Ansprüche 1 bis 8, **dadurch gekennzeichnet, dass** das Collagen in einer Konzentration im Bereich von 2 bis 6 mg/ml und bevorzugt im Bereich von 3 bis 5 mg/ml vorliegt.

10. Verfahren nach einem der Ansprüche 1 bis 9, **dadurch gekennzeichnet, dass** das Glycationsmittel ein Mittel ist, das die Glycation erlaubt, d. h., das befähigt ist, nach der Maillard-Reaktion mit einer Aminogruppe des Collagens unter Bildung einer Schiffschen Base zu reagieren.

11. Verfahren nach Anspruch 10, **dadurch gekennzeichnet, dass** das Glycationsmittel unter Glucoson, 3-Desoxyclucoson, Glyoxal, und Methylglyoxal oder auch Zuckern ausgewählt wird.

12. Verfahren nach Anspruch 11, **dadurch gekennzeichnet, dass** das Glycationsmittel ein Zucker ist.

13. Verfahren nach Anspruch 12, **dadurch gekennzeichnet, dass** der Zucker unter den Osen ausgewählt wird.

14. Verfahren nach Anspruch 13, **dadurch gekennzeichnet, dass** die Ose unter Ribose, Fructose oder Glucose ausgewählt wird.

15. Verfahren nach einem der Ansprüche 1 bis 14, **dadurch gekennzeichnet, dass** das Glycationsmittel unter Ribose oder Glucose ausgewählt wird.

16. Verfahren nach einem der Ansprüche 1 bis 15, **dadurch gekennzeichnet, dass** die Menge an Glycationsmittel im Bereich vom 0,5 bis 20 Gew.-% und bevorzugt im Bereich vom 1 bis 10 Gew.-% liegt, bezogen auf das Gesamtgewicht der Collagenlösung.

17. Verfahren nach einem der Ansprüche 1 bis 16, **dadurch gekennzeichnet, dass** die Glycationsreaktion bei einer Temperatur im Bereich von 15 bis 30 °C und bevorzugt im Bereich von 20 bis 25 °C vorgenommen wird.

18. Verfahren nach einem der Ansprüche 1 bis 17, **dadurch gekennzeichnet, dass** die Dauer der Glycationsreaktion 15 Tage bis 2 Monate und bevorzugt 25 bis 35 Tage beträgt.

19. Verfahren nach einem der Ansprüche 1 bis 18, **dadurch gekennzeichnet, dass** das Verhältnis von glykiertem Collagen zu nichtglykiertem Collagen im Bereich von 25 bis 75 und bevorzugt im Bereich von 45 bis 55 liegt.

20. Verfahren nach einem der Ansprüche 1 bis 19, **dadurch gekennzeichnet, dass** die gemäß der Erfindung verwendeten Keratinocyten menschlichen Ursprungs sind.

21. Äquivalent der Alters-Dermis, das nach dem Verfahren nach einem der Ansprüche 1 bis 20 erhältlich ist und mindestens glykiertes Collagen und Fibroblasten aufweist, **dadurch gekennzeichnet, dass** es einen Glycationsgrad von 2 bis 30 aufweist.

22. Äquivalent der Alters-Dermis nach Anspruch 21, **dadurch gekennzeichnet, dass** es einen Glycationsgrad von 8 bis 18 aufweist.

23. Äquivalent der Alters-Dermis nach einem der Ansprüche 21 oder 22, **dadurch gekennzeichnet, dass** das glykierte Collagen tierischen oder menschlichen Ursprungs ist.

24. Äquivalent der Alters-Dermis nach einem der Ansprüche 21 bis 23, **dadurch gekennzeichnet, dass** das glykierte Collagen tierischen Ursprungs ist.

25. Äquivalent der Alters-Dermis nach einem der Ansprüche 21 bis 24, **dadurch gekennzeichnet, dass** das glykierte Collagen ein Rindercollagen ist.

26. Äquivalent der Alters-Dermis nach einem der Ansprüche 21 bis 25, **dadurch gekennzeichnet, dass** das glykierte Collagen vom Typ I ist.

27. Äquivalent der Alters-Dermis nach einem der Ansprüche 21 bis 26, **dadurch gekennzeichnet, dass** die Fibroblasten menschlichen Ursprungs sind.

28. Äquivalent der Epidermis, das zumindest Keratinocyten enthält, **dadurch gekennzeichnet, dass** es erhältlich ist durch Einsäen zumindest von Keratinocyten auf ein Äquivalent der Dermis wie in einem der Ansprüche 21 bis 27 definiert, sowie **dadurch**, dass es in den Zellen zumindest der drei ersten suprabasalen Schichten Integrin β1 exprimiert.

29. Äquivalent der Epidermis nach Anspruch 28, **dadurch gekennzeichnet, dass** die Keratinocyten menschlichen Ursprungs sind.

30. Äquivalent der Epidermis nach einem der Ansprüche 28 oder 29, **dadurch gekennzeichnet, dass** es ferner Melanocyten und/oder Langerhans-Zellen und/oder Vorläufer von Langerhans-Zellen aufweist.

31. Äquivalent der Altershaut, **dadurch gekennzeichnet, dass** es mindestens ein Äquivalent der Epidermis und ein Äquivalent der Alters-Dermis wie in einem der Ansprüche 21 bis 27 beschrieben umfasst.

32. Äquivalent der Altershaut nach Anspruch 31, **dadurch gekennzeichnet, dass** es ein Äquivalent der Epidermis wie in einem der Ansprüche 28 bis 30 beschrieben umfasst.

33. In-vitro-Verwendung eines Äquivalents der Alters-Dermis wie in einem der Ansprüche 21 bis 27 beschrieben zur Herstellung eines Äquivalents der Epidermis und/oder der Altershaut.

34. In-vitro-Verwendung eines Äquivalents der Alters-Dermis wie in einem der Ansprüche 21 bis 27 beschrieben zur Untersuchung des Phänomens der Glycation selbst sowie von Modulatoren (Inhibitoren oder Aktivatoren) dieses Phänomens, zur Untersuchung der Lichtalterung und der Wirkung ultravioletter Strahlung auf die Dermis sowie von Modulatoren dieser Wirkungen (Verbindungen und/oder Zusammensetzung zum Schutz, Filter, etc.) oder zur Untersuchung des Einflusses der Glycation auf die Bestandteile der Dermis.

35. In-vitro-Verwendung eines Äquivalents der Epidermis wie in einem der Ansprüche 28 bis 30 beschrieben zur Untersuchung des Phänomens der Glycation selbst sowie von Modulatoren (Inhibitoren oder Aktivatoren) dieses Phänomens, zur Untersuchung von mit der Alters-Epidermis in Verbindung stehenden Phänomenen wie z. B. Falten, zur Untersuchung von Modulatoren (isolierte Verbindungen und/oder Zusammensetzungen) des Auftretens von Falten (insbesondere Inhibitoren), zur Untersuchung der Lichtalterung und der Wirkung ultravioletter Strahlung auf die Epidermis sowie von Modulatoren dieser Wirkungen (Verbindungen und/oder Zusammensetzungen zum Schutz, Filter, etc.) oder zur Untersuchung des Einflusses der Glycation auf die Bestandteile der Epidermis und der Hautanhangsgebilde.

36. In-vitro-Verwendung eines Äquivalents der Altershaut wie in Anspruch 31 oder 32 beschrieben zur Untersuchung des Phänomens der Glycation selbst sowie von Modulatoren (Inhibitoren oder Aktivatoren) dieses Phänomens, zur Untersuchung der mit der Altershaut und/oder der Alters-Epidermis in Verbindung stehenden Phänomene wie z. B. Falten, zur Untersuchung von Modulatoren (isolierte Verbindungen und/oder Zusammensetzungen) des Auftretens von Falten (insbesondere Inhibitoren), zur Untersuchung der Lichtalterung und der Wirkung ultravioletter Strahlung auf die Haut und/oder die Dermis und/oder die Epidermis sowie von Modulatoren dieser Wirkungen (Verbindungen und/oder Zusammensetzungen zum Schutz, Filter, etc.), zur Untersuchung des Einflusses der Glycation auf die Bestandteile und/oder die Anhangsgebilde der Haut und/oder der Dermis und/oder der Epidermis (Körperhaare und/oder Kopfhaare, Blutgefäße, Nervenfasern, etc.) sowie auf therapeutischem Gebiet zur Untersuchung von durch Diabetes hervorgerufenen Komplikationen über die Glycation.
